# EUROPEAN PATENT SPECIFICATION

(11) **EP 2 776 014 B1**
(45) Date of publication and mention of the grant of the patent: **01.07.2026**
(21) Application number: 12790837.4
(22) Date of filing: 09.11.2012
(51) Int. Cl.: A61K 9/14, A61K 9/16, A61K 31/4045, A61K 9/00

(54) **MELATONIN-BASED SOLUTIONS AND POWDERS FOR THEIR PREPARATION**
AUF MELATONIN BASIERENDE LÖSUNGEN UND PULVER ZU IHRER HERSTELLUNG
SOLUTIONS À BASE DE MÉLATONINE ET POUDRES POUR LEUR PRÉPARATION

(30) Priority: 10.11.2011 IT MI20112042
(43) Date of publication of application: 17.09.2014
(73) Proprietor: Zambon S.p.A., 20091 Bresso (MI) (IT)
(72) Inventor: MAGGI, Loretta, I-29100 Piacenza (IT); CAPONETTI, Giovanni, I-29100 Piacenza (IT)
(74) Representative: Giavarini, Francesco
(86) International application number: PCT/EP2012/072327
(87) International publication number: WO 2013/068565

(56) References cited:
- EP-A1- 1 174 134
- WO-A1-02/076452
- WO-A1-2010/062153
- WO-A1-2011/150098
- WO-A2-2005/062992
- CN-A- 1 689 422
- CN-A- 1 969 814

## Description

The present invention relates to a powder for reconstitution before use for preparations of a solution comprising melatonin. More in particular, the present invention relates to a powder for reconstitution before use for preparations of a solution, comprising melatonin for use in the treatment of cerebral infarction. Melatonin-based solutions are also part of the invention. Melatonin is a hormone secreted by the pinealocytes of the pineal gland, or pinealocytes, during the night, with a 24-hour circadian rhythm trend, characterised by very low levels during the day and an increase at night that starts at around 8.00 p.m. and reaches peak values between 2 and 4 in the morning.

In plasma, the concentration of melatonin, which circulates bound to albumin, varies in the range 10 - 300 pg/ml.

Its half-life is short (30-60 minutes), due to the 90% clearance following its first passage through the liver. Approximately 75% of the melatonin metabolised by the liver cells is converted into 6-hydroxymelatonin, then conjugated with sulphate (70%) and, to a lesser extent, with glucuronic acid (6%). Bioavailability is low and equal to approximately 15%. Melatonin appears to be rapidly absorbed if administered as oral solutions, and the peak blood concentration is the highest of those reported for similar doses in healthy individuals. After oral administration, its peak blood concentration (Cmax) is influenced by the solubility of the melatonin in the formulation, bioavailability alterations and clearance.

In the past melatonin has been studied for pharmaceutical purposes, by developing preparations for oral administration. These preparations include formulations with cyclodextrin and as microemulsions. Nevertheless, and common to many other preparations, it may be necessary to wait longer than 30 minutes from administration to achieve peak blood concentration of melatonin. This is due in part to the need for gastrointestinal absorption to be complete, for the melatonin to be available in the bloodstream. Moreover, the bioavailability of melatonin is low and very variable. The absolute availability of melatonin via the oral route was shown to be approximately 15% due to a significant effect of a first hepatic passage, and peak plasma concentrations can vary by as much as almost 20-fold. For this reason, the oral administration of melatonin through the currently available formulas does not provide a rapid onset of action and the variable absorption makes this route of administration impractical.

The increase in blood melatonin levels, achieved by taking both physiological and pharmacological doses, favours the induction of sleep, as well as its duration and quality, providing a well-documented hypnotic action, similar to that of benzodiazepines.

Thanks to its hypnotic action, melatonin has been proposed as the main agent or as a coadjuvant for many applications including conditions that are typically related to the paediatric age such as:
- dyssomnias and difficulties initiating and maintaining sleep. Among these, delayed sleep-phase syndrome (DSPS) and advance sleep-phase syndrome (ASPS).
- Neurological impairments that affect irregular sleep-wake patterns such as: mental or intellectual disabilities, mental retardation, learning disabilities, autistic spectrum disorders, Rett syndrome, tuberous sclerosis, developmental disabilities and Angelman syndrome.

In addition, melatonin has been evaluated for its usefulness in the treatment of sleep trouble of Autism Spectrum Disorders (ASDs).

Sleep problems including delayed sleep onset, sleep or bedtime resistance, prolonged tiredness upon waking and daytime sleepiness as well as Attention Deficit Hyperactivity Disorder (ADHD), Smith Magenis Syndrome (SMS) and Sanfilippo Syndrome (SFS) can also be treated using melatonin.

Other potentially relevant uses of melatonin are related to the premedication preceding the anesthesia induction.

The evidence reported that patients with seizures of diverse origin show an alteration of the melatonin rhythm is supportive of its use also for this application.

Since newborns and particularly those delivered preterm have less protection against oxidation and are highly susceptible to free radical-mediated oxidative damage, melatonin, because of its antioxidant properties could be useful to reduce oxidative stress in neonates with sepsis, asphyxia, respiratory distress or surgical stress.

This anti-radical action is synergic to that of vitamin E, which protects the entire cell from oxidative stress with various mechanisms, including the boosting of enzyme systems such as glutathione peroxidase and superoxide dismutase. In vitro experiments have definitively confirmed the inhibiting effect of melatonin on lipid peroxidation with a synergic effect to that of retinoids.

It is well documented that melatonin exhibits a circadian rhythms in body fluids, and human milk is no exception. Melatonin in the milk of lactating mothers exhibits marked daily rhythm with high levels during the night and undetectable levels during the day. This melatonin rhythm in milk could serve to communicate time of day information to breast-fed infants, this information could also contribute to the consolidation of sleep-wake rhythm of infants until the maturation of their own circadian rhythm.

Melatonin plays an important role as a scavenger of both reactive oxygen species and reactive nitrogen species, including peroxynitrite (ONOO-). Moreover, it increases the activity of various antioxidant enzymes such as superoxide dismutase (SOD) or glutathione peroxidase (GSH-Px) and it induces the activity of gamma-glutamylcysteine synthetase thereby stimulating the production of another antioxidant, glutathione (GSH). Melatonin not only regulates the activity of these enzymes, but also their mRNA levels.

In addition to the above mentioned characteristics, melatonin is considered an important antioxidant as it is a lipophilic and hydrophilic molecule and able to easily cross all biological barriers, including the blood-brain barrier. Moreover, it is available in all bodily tissues and cells, distributing itself to all the cell parts, but primarily in the nucleus and mitochondria.

Thanks to the antioxidant characteristics mentioned above, melatonin can be used advantageously in the treatment of cerebral infarction and cerebral paralysis. The term "cerebral infarction" is intended as an infarction of any tissue or part of the brain. Cerebral infarction is caused by cerebral ischaemia or hypoxaemia. As is well known, the main causes of cerebral ischaemia are cerebral thrombosis and cerebral embolism.

For the treatment of these events, melatonin has been positively studied in form of a micro-encapsulated system, such as the liposomal form.

The advantages of this administration are related to the possibility of releasing the melatonin into the bloodstream gradually so that its apparent residence time therein increases and leads to an increase in the same bioavailability of the active ingredient.

Other examples of encapsulated systems proposed for melatonin are related to the creation of cyclodextrin matrices or to the use of synthetic or natural biodegradable polymers such as polylactic acid or copolymers of lactic acid and glycolic acid.

CN1969814A discloses a solution comprising a low amount of melatonin, at least one soluble excipient, at least a surfactant, water and a polyalkylene glycol.

WO2005/062992A2 discloses a powder compositions comprising a very low amount of melatonin, at least one water soluble excipient (albumin) and at least one water soluble surfactant, having an X90 of less than 100 micron and a VDM of less than 50 micron.

EP1174134B1 describes a pharmaceutical or dietary composition for the treatment of cerebral infarction. Said pharmaceutical composition is administered via the oral route, in order to reduce the effects of the infarction. However, this type of administration presents a number of limits, since modest blood concentrations of melatonin are obtained due to its rapid hepatic metabolism. Consequently, low levels of the medicinal product are able to cross the blood-brain barrier and reach the damaged brain areas. Moreover, due to its poor solubility, a significant portion of the dose administered via the oral route is swallowed undissolved in saliva and is responsible for the low and variable bioavailability of melatonin via the gastrointestinal route. Melatonin is, in fact characterised by poor solubility in a solvent medium that is constituted primarily by water.

This poor solubility has significantly restricted its use since, despite having a high anti-oxidant pharmacological activity, the doses needed to obtain this kind of activity must be quite high.

Thanks to its antioxidant activity, melatonin could be successfully used in the prophylaxis of certain neurological diseases and to prevent acute cerebral events, however the necessity to administer quite high doses restricts its use, as does the impossibility of being able to develop liquid formulations in which to dissolve a large amount in a small volume.

As a consequence, melatonin is currently commercially used only in nutraceutical or "over-the-counter" products for the symptomatic treatment of jetlag. This syndrome is, in fact, treated with oral administration of 3 mg tablets without requiring any particular industrial processing. However, in the case of the use of melatonin as an antioxidant agent, although significant scientific evidence exists to support this pharmacological effect, at the current time there are no products available on the market containing melatonin with an antioxidant function.

To demonstrate the difficulties of developing formulations containing melatonin for antioxidant use, a number of patents mention the possibility of using melatonin at high doses but do not describe the procedure used for the preparation of such doses.

More in particular, in the case of the administration of melatonin to a premature newborn, there is a conflict between the difficulty to reconcile requirements such as the administration of a high dose of melatonin and the impossibility of injecting into this particular patient a volume of liquid too large.

With respect to the administration of a pharmaceutical agent in a paediatric setting, and more specifically to a preterm child, 3 type of formulations are to be considered acceptable:
- oral solutions
- injectable solutions
- rectal solutions.

When considering oral administration, oral liquid dosage forms would normally be considered acceptable for children from full term birth.

According to the Reflection paper: formulations of choice for the paediatric population (EMEA/CHMP/PEG/194810/2005), typical target dose volumes for paediatric liquid form

| Age | Volume (ml) |
|---|---|
| Preterm newborn infants | 2 |
| Term newborn infants (0d-28d) | 4 |
| Infants and toddlers (1m-2y) | 5 |
| Children (pre school) (2-5y) | 5 |
| Children (school) (6-11y) | 4 |
| Adolescents (12-16/18y) | 4 |

Based on the table presented, it appears very clear that the only applicable approach for paediatric administration of oral solutions is significantly limited by the acceptable volume of delivery. Especially when dealing with low solubility active principles.

Nevertheless an oral liquid dosage form will normally need to be preserved. It is very well documented that preservatives have a potential to cause toxicological problems, especially in young children.

As an alternative to the oral route, the rectal route of administration can be utilized to achieve a systemic effect in children. Also immediate systemic effects can be achieved as documented by the administration of diazepam to resolve epileptic seizures.

Also in this case volume limitations have to be taken into consideration.

Typical target dose volumes for paediatric liquid enemas are presented in the table here below:

| Age | Volume (ml) |
|---|---|
| Preterm newborn infants | 5 |
| Term newborn infants (0d-28d) | 4 |
| Infants and toddlers (1m-2y) | 4 |
| Children (pre school) (2-5y) | 3 |
| Children (school) (6-11y) | 3 |
| Adolescents (12-16/18y) | 2 |

The volume of the enemas is related to its function and to the age of the child. Nevertheless, volumes of enemas for systemic therapy in paediatric patients should be as small as possible to achieve accurate delivery, good absorption and absence of irritation.

The solutions can be administered as they are or eventually their viscosity can be increased with the addition of a thickening agent like a cellulose polymer to form a gel.

Excipients used in rectal dosage forms should not irritate the rectal mucosa of infants and children.

Also parenteral formulations are suitable for paediatric administration.

The most appropriate method on injection for emergency practices are to be considered the intravenous injection and the infusion.

For seriously ill paediatric patients the intravenous route is preferred and if possible, injections are administered through an indwelling venous cannula.

Both for i.v. solutions and for pump systems, volume limitations are to be considered according to the table here below:

| Age | Volume (ml) |
|---|---|
| Preterm newborn infants | 5 |
| Term newborn infants (0d-28d) | 4 |
| Infants and toddlers (1m-2y) | 4 |
| Children (pre school) (2-5y) | 4 |
| Children (school) (6-11y) | 4 |
| Adolescents (12-16/18y) | 3 |

The venous access may be by a small cannulae in a peripheral vein.

Peripheral veins with slow blood flow will be irritated by a high osmotic load, extremes of pH and the chemical nature of some active substances and excipients. Phlebitis, thrombo-phlebitis or infiltration of the tissue may result with loss of the vein for therapy and possibly tissue damage.

With respect to osmoticity, this characteristic of the formulation has special importance in the i.v. infusion of highly concentrated nutritional solutions.

The hyperosmoticity problem is generally solved by injecting the solution centrally into a large volume of rapidly moving blood, instead of using peripheral infusions.

Use of such solutions and knowledge of their value have led to the use of similar formulations administered, not parenterally but orally. Nevertheless, when certain solutes are ingested in large amounts or as concentrated fluids, their osmotic characteristics can cause and upset in the normal water balance within the body.

In these cases, when highly osmotic solutions are ingested, large amounts of water will transfer to the stomach and intestines from the fluid surrounding those organs, in an attempt to lower the osmoticity.

The higher the osmoticity, the larger the amount of water required. A large amount of water in the GI tract can cause distention, cramps, nausea, vomiting, hypermotility and shock.

Hyperosmoticity feeding may result in mucosal damage in the GI tract following both oral and rectal administration..

With respect to the preparation of a solution for injectable, oral or rectal administration another critical aspect of these preparation relates to stability of melatonin in solution.

Good evidences are reported that indicate that melatonin solutions gradually lose potency at all pH values and are not stable when exposed to light or oxygen.

It would therefore be necessary to develop, for this therapy, a melatonin formulation at a concentration of at least 5 mg/ml, which is not toxic and can be administered intravenously to a premature newborn.

The development of aqueous solutions with a high melatonin concentration was tackled following different approaches.

The first approach adopted was that of dissolution in a water-alcohol solution in which ethanol made up as much as 35% of the total volume. It is therefore obvious that this approach is not compatible with a treatment intended for a premature infant.

The simplest solution adopted, on the other hand, was to completely avoid using ethanol and to use in replacement of the same, as a co-solvent, a glycol to be mixed with water.

This approach would make it possible, in theoretical terms, to obtain even quite concentrated solutions of melatonin without using toxic organic solvents.

US5939084 describes compositions containing melatonin for both pharmaceutical and cosmetic use, in solutions of water and PEG at different concentrations.

However, the proposed approach is not without contraindications, since melatonin thus formulated was not stable either in quantitative terms, with loss of content, or in qualitative terms, with the development of degradation products.

In addition, due to the presence of isoprene glycol, butylene glycol or propylene glycol in the composition the osmolality of the solutions presented don't have an acceptable level of osmolality for an injectable application or an oral and rectal administration
As regards the melatonin stability in aqueous solution, it is acknowledged by literature *(*Daya S., Walker RB, Glass BD, Anoopkumar-Dukie S., The effect of variations in pH and temperature on stability of melatonin in aqueous solution; J. Pineal Res. 2001; 31:155-158) that aqueous solutions of this active substance present stability problems. The melatonin content in aqueous solutions at a concentration of 50 µg/ml and a pH of 7.4 show, after just 5 days, a loss in melatonin content of 9% and 10% when stored at 20°C and 37°C, respectively.

Moreover, with the same solution, at the pH values 4.0 and 7.4 a loss of melatonin potency of 22% or 29% respectively was observed upon storage at 20°C.

A degradation of this extent in such a short time is clearly incompatible with the development of a product in solution form whose stability should be guaranteed for at least 6 months. Moreover there is a risk, which has been reported in certain cases, that melatonin in solution may partially recrystallise with precipitation adding to the risk of a loss of active substance content also that of administrating to the patient an intravenous solution containing suspended particles.

Consequently, from this point of view, storing melatonin solutions at a low temperature (e.g. 4°C) to maintain their chemical stability could compromise the preparation's physical stability. We have surprisingly found that it is possible to retain melatonin stability at concentrations higher than 2 mg/ml together with solution osmolality acceptable for injection application or oral and rectal administration.

In order to guarantee both the chemical and physical stability of the melatonin in the solution, the most suitable technological approach would be to make up melatonin solutions extemporaneously, to be administered at the time of use.

However, this option is precluded in the case in which pure melatonin in a crystalline form is to be used, since it has a low dissolution speed both in aqueous solutions and in water and PEG solutions.

Melatonin's low dissolution speed represents the second important problem concerning the preparation of high concentration aqueous solutions for administration by injection.

In the case of injection therapy in newborns, the issues of administering high doses of melatonin are heightened by the patient's inability to cooperate in the administration of the medicinal product and, above all, the minimum plasma volumes, which restrict the volume of liquid that can be injected.

To this we can also add the need for a rapid formulation dissolution with a reduction in the size of the formulate that must be present in the form of a clear solution.

Given this need, any dry melatonin micronisation technique is clearly precluded as the product that can be obtained by this route would be in crystalline form and therefore very slow to dissolve.

It would therefore be preferable to have a stable melatonin formulation, in order to obtain a therapeutically acceptable dose.

It would, moreover, be preferable to have a pharmaceutical formulation for injection able to satisfy the injectability criteria for medicinal products and that it is therefore devoid of substances that are harmful or toxic for the organism.

It would also be preferable to dispose of a pharmaceutical melatonin liquid formulation in form of solution, for the treatment of neonatal conditions, therefore with limited injected or administered volumes in order to avoid generating imbalances in the blood volumes of the patients treated.

Thus a general aim of the invention is to provide melatonin solution for injectable, rectal or oral preparations with acceptable values of pH and osmoticity of this preparation.

According to this invention such general aim is obtained by a pharmaceutical composition in the form of a solution characterised by comprising melatonin in an amount from 2mg/ml to 20mg/ml, said solution having an osmolality of less than 1400 mOsm/kg, preferably of less than 900 mOsm/kg more preferably from 300 mOsm/kg to 700 mOsm/kg.

According to this invention the osmolality is defined as the concentration of a solution in terms of osmoles of solute per kilogram of solvent. It is expressed in terms of Osm/kg or mOsm/kg. Another aim of the present invention is to provide a stable and easy to administer melatonin formulation, with adequate concentrations of active substance, such as to be able to reach therapeutically acceptable doses.

A further aim of the present invention is to provide a melatonin formulation for injection that respects the requirements envisaged for such form of administration and that is therefore devoid of toxic substances or those not compatible with parenteral or intravenous administration.

Yet another aim of the present invention is to provide a melatonin formulation for injection for the treatment of neonatal conditions that is compatible with such subjects and presents reduced administration volumes.

According to this invention, the above mentioned aims are achieved by means of a powder for use as a medicament, containing melatonin in an amounts from 35 to 90% in weight, at least one water soluble excipient in an amount from 5 to 60% in weight wherein said soluble excipient comprises leucine and at least one water soluble surfactant in an amount from 0.5 to 5% of the total weight of the powder, said powder having an X90 of less than 100 µm and a volume mean diameter VDM of less than 50 µm; and wherein said powder is obtained by spray drying from a solution comprising melatonin.

According to this invention X90 is the limit values of 90% of the dimensional distribution of the powder, and VMD is the volume mean diameter of the powder.

A further aim of the present invention concerns a composition in the form of a solution obtained by dissolving a powder containing melatonin in an amounts from 35 to 90% in weight, at least one water soluble excipient in an amount from 5 to 60% in weight and at least one water soluble surfactant in an amount from 0.5 to 5% of the total weight of the powder, said powder having an X90 of less than 100 µm and a VMD of less than 50 µm, in a mixture of water and polyalkylene glycol, in which melatonin is present in an amounts from 2 to 30 mg/ml and the polyalkylene glycol is present in an amounts from 5 to 40% of the total volume of the liquid used. According to this invention X90 and VMD are the same as described above.

Another aspect of the present invention relates to a kit for the extemporaneous preparation of a solution for injection, including: a powder containing melatonin in an amounts from 35 to 90% in weight, at least one water soluble excipient in an amount from 5 to 60% in weight and at least one water soluble surfactant in an amount from 0.5 to 5% of the total weight of the powder, said powder having an X90 of less than 100 µm and a VDM of less than 50 µm, and a liquid medium comprising H₂O and polyalkylene glycol in quantities from 5 to 40% of the total volume of the liquid used.

Preferably, in order to obtain a pharmaceutical form of melatonin that can be used in the treatment of cerebral infarction and in particular in neonatal cerebral infarction, the concentration of melatonin in the pharmaceutical form is from 2mg/ml to 20 mg/ml, preferably from 5 mg/ml to 15 mg/ml more preferably from 8 mg/ml to 12 mg/ml.

The solution according to the present application it is also suitable for using in the treatment of prevention of perinatal asphyxia, neonatal cerebral infarction, treatment or sleep disorders in a pediatric patient, treatment of sleep disorders in Autism Spectrum Disorders (ASD) and for use in preanesthesia.

The preferred production process for the powder for reconstitution according to the present invention is that of spray drying using a water-alcohol solution of a soluble excipient and a surfactant in which the melatonin is dissolved or dispersed as a suspension or emulsion.

With the spray drying technique it is possible to obtain particles containing both melatonin and the soluble excipient. The preferred morphology is an internally hollow one, in order to obtain particles with a reduced density when poured onto the bed of powder; in this way the melatonin is dispersed in said soluble excipient or combined and interpenetrated with it.

From the point of view of stability, the melatonin is indeed entrapped or combined and interpenetrated into a matrix that includes all the excipients that at the same time guarantee the stability of the powder preparation during its handling and storage, thereby avoiding the need to keep the product in controlled conditions of temperature and/or humidity.

The soluble excipient or excipients present in the powder for reconstitution according to the present invention usually have a solubility in water greater than 5 mg/ml and often greater than 100 mg/ml or over. They are preferably chosen from sugars, salts, amino acids and certain soluble polymers and can perform multiple functions, such as:
- facilitating the rapid dissolution of the dry composition;
- favouring the formation of particles with a small diameter during preparation for spray drying;
- improving the chemical and physical stability of the melatonin in the powder formulation;
- making sure that the melatonin can be kept in a preferred crystalline or amorphous solid state;
- improving the hydrophobicity of the surface of the particles containing melatonin, protecting it from any harmful effects of environmental humidity;
- favouring the fluidity of the powder containing melatonin.

As regards the composition in its dry solid form, the excipient constitutes the structural element for the formation of a solid matrix, inside which the melatonin is interpenetrated. Examples of soluble excipients that can be used in the composition according to the present invention are: alitame, acesulfame potassium, aspartame, saccharin, sodium saccharin, sodium cyclamate, sucralose, trehalose, xylitol, citric acid, tartaric acid, cyclodextrin, dextrin, hydroxy ethyl cellulose, gelatine, malic acid, maltitol, maltodextrin, maltose, polydextrose, tartaric acid, sodium or potassium bicarbonate, sodium or potassium chloride, sodium or potassium citrate, phospholipids, lactose, sucrose, glucose, fructose, mannitol, sorbitol, natural amino acids, alanine, glycine, serine, cysteine, phenylalanine, thyroxin, tryptophan, histidine, methionine, threonine, valine, isoleucine, leucine, arginine, lysine, aspartic acid, glutamic acid, asparagine, glutamine, proline, their salts and any simple chemical modifications as in the case of N-acetyl cysteine and carbocysteine.

The preferred soluble excipients are the salts of alkali metals such as sodium chloride or potassium chloride and sugars such as lactose.

Examples of preferred soluble polymers are hyaluronic acid of any molecular weight, its salts and derivatives.

As far as the hollow morphology of the particles is concerned, the composition requires the presence of a soluble excipient, preferably a sugar such as lactose, able to form instantaneously in the solvent evaporation stage during spray drying, the skeleton of the particle and thereby producing particles with a high porosity.

The surfactant present in the powder for reconstitution according to the present invention can be chosen from various classes of surfactants for pharmaceutical use.

Surfactants to be considered suitable for use in the present invention are all those substances characterised by a medium or low molecular weight that contain a hydrophobic portion, which is generally readily soluble in an organic solvent but poorly soluble or completely insoluble in water, and a hydrophilic (or polar) portion, which is slightly soluble or completely insoluble in an organic solvent but readily soluble in water. Surfactants are classified according to their polar portion; therefore, surfactants with a negatively charged polar portion are defined anionic surfactants whereas cationic surfactants contain a positively-charged polar portion. Uncharged surfactants are general defined non-ionic, whereas the surfactants that contain both a positively charged group and a negatively charged group are defined zwitterionic. Examples of anionic surfactants are fatty acid salts (better known as soaps), sulphates, sulphate ethers and phosphate esters. Cationic surfactants are frequently based on polar groups containing amine groups. The most common non-ionic surfactants are based on polar groups containing oligo-(ethyleneoxide) groups. Zwitterionic surfactants are generally characterised by a polar group constituted by a quaternary amine and a sulphuric or carboxylic group.

The following surfactants are examples of this application: benzalkonium chloride, cetrimide, docusate sodium, glyceryl monooleate, sorbitan esters, sodium lauryl sulphate, polysorbates, phospholipids and bile salts.

Non-ionic surfactants such as polysorbates and polyoxyethylene and polyoxypropylene block copolymers known as "Poloxamers" are preferred. Polysorbates are described in the CTFA International Cosmetic Ingredient Dictionary as mixtures of sorbitol fatty acid esters and sorbitol anhydrides condensed with ethylene oxide. Non-ionic surfactants belonging to the "Tween" series are particularly preferred, especially the surfactant known as "Tween 80", a commercial monooloeate polyoxyethylene sorbitan, other preferred surfactants are: polyoxyethylene alkyl ethers (also known with the trade name Brij), ricin oil polyoxyethylene ethers (known with the trade name Cremophor), polyoxyethylene stearates (known as PEG stearates) glyceryl monooleate and glyceryl monostearate (known with the trade name Tegin or Myverol).

The presence of a surfactant, and preferably, Poloxamer 188 is necessary to guarantee the abatement of electrostatic charges, maintenance of the powder's fluidity and maintenance of the solid state in a homogeneous way, without initial crystallisation.

The powder for reconstitution according to the present invention can comprise other components, such as pH buffers and preservatives, however such components are generally not essential due to the fact that the composition is stored in a dry solid form and the relative aqueous solution is prepared extemporaneously before use.

The process for preparation of the powder for reconstitution according to the invention substantially comprises the operations of:
a) preparing a first phase (a) in which the melatonin is present in a suitable liquid medium;
b) preparing a second phase (b) in which soluble excipients are dissolved and surfactants are dissolved or dispersed in an aqueous medium;
c) mixing said phases (a) and (b) to obtain a third phase (c) in which the liquid medium is homogeneous;
d) drying said phase (c) in controlled conditions to obtain a dry powder with particles whose dimensional distribution has a median diameter of less than 50 µm and an X90 of less than 100µm;
e) collecting said dry powder and packaging it in a form suited to the extemporaneous preparation of a solution.

Operation d) consists in eliminating the liquid medium, solvent or dispersant, from phase (c), to obtain a dry powder having the desired dimensional characteristics. Such drying shall be preferably obtained by spray drying. The characteristics of the nozzle and the parameters of the operation are chosen so that the liquid medium is evaporated from solution or suspension (c) and a powder with the desired particle characteristics forms.

The term powder for reconstitution according to the present invention is intended as a powder used for the extemporaneous preparation of a stable solution of melatonin for injection in a suitable volume of water or sterile saline solution.

As is known in the field, the extemporaneous preparation is made up at the time of use, i.e. immediately before administration of the medicinal product to the patient. In this description, the term "extemporaneous preparation" also includes the preparation made up by a pharmacist and destined to be administered to a patient within a relatively short period of time from preparation. More in general, the term "extemporaneous preparation" is used to designate all those cases in which the solution is not manufactured directly by the pharmaceutical company and marketed as such to be used, rather to be prepared at a time subsequent to that in which the dry solid composition is manufactured, usually a time close to the time of administration to the patient.

In particular, the powder for reconstitution is dissolved in a mixture of water and polyalkylene glycol in which the polyalkylene glycol is present in a quantity from 5 to 40% of the total volume, preferably in a quantity from 10% to 30%, such as to obtain a preparation for injection in the form of a solution containing melatonin.

In order to obtain a therapeutically acceptable administration, the melatonin in the preparation for injection obtained by dissolution of the powder for reconstitution according to the present invention, is present in quantities from 2 to 30 mg/ml, preferably in a concentration from 5 to 15 mg/ml.

The polyalkylene glycol present in the dissolution medium for the powder according to this invention advantageously contributes to the dissolution of the melatonin in water, such as to obtain higher concentrations of the active substance in the solution for injection, in particular, the preferred polyalkylene glycol is polyethylene glycol (PEG) with a molecular weight from 200 to 600.

In order to achieve a rectal preparation, a thickening or gelling agent is added in the solution in amount from 0,5% to 50.0% w/v of the composition: Examples of soluble thickening or gelling agent that can be used in the composition according to the present invention are: carboxymethylcellulose (CMC), hydroxypropylmethylcellulose (HPMC), hydroxyethylcellulose, hydroxypropylcellulose, microcrystalline cellulose (Avicel), chitosan, Sodium Alginate, Alginic acid, carrageenan, Guar gum, Gelatin, Hyrpomellose, Polyvinylpirrolidone (PVP), Poloxamers, Polyethylene glycols (MW > 600).

With regard to the oral administration, it is possible to mix the solution according to the present invention with breast feed milk, in order to achieve an oral administration for neonatal or for children

The invention shall now be illustrated with reference to the following non-limiting examples.

### EXAMPLES

### PREPARATION OF THE POWDER FOR RECONSTITUTION

Powders containing melatonin were obtained according to the compositions indicated in Table 1, which also indicates the quantities of dried product and volumes of solution produced. Examples 1 and 2 concern the preparation of reference powders constituted by pure melatonin produced by spray drying and from melatonin with addition of the surfactant alone, without using excipients.

All the preparations in the examples were implemented and characterised in standard environmental conditions of 25°C and 50% RH (relative humidity).

For the powder preparations, in all cases the melatonin was first dissolved in ethanol and the excipients dissolved in water. The two solutions thus obtained were combined slowly at an ambient temperature of 25°C, taking care not to cause the precipitation of any of the components. The water-alcohol solution thus obtained was processed using a Buchi model B290 spray drier with condenser and recirculating closed circuit system.

The instrument was fitted with a nozzle with a diameter of 0.5mm.
Atomization gas: nitrogen
Inlet temperature: 150°C
Nebulisation pressure: 2.5 bar
Powder collection system: cyclone separator coupled with a nylon filter sleeve.

**Table 1**

| **Example** | **Composition** | **%** | **Quantity (g)** | **Solvent** |
|---|---|---|---|---|
| 1 - (comp.) | Melatonin | 100.0 | 50.0 | 500 ml EtOH |
| | | | | 500 ml Water |
| 2 - (comp.) | Melatonin | 99.0 | 49.5 | 500 ml EtOH |
| | Tween 80 | 1.0 | 0.5 | 500 ml Water |
| 3 | Melatonin | 50.0 | 25.0 | 500 ml EtOH |
| | Lactose | 49.0 | 24.5 | |
| | Tween 80 | 1.0 | 0.5 | 500 ml Water |
| 4 | Melatonin | 70.0 | 35.0 | |
| | Mannitol | 24.0 | 12.0 | 500 ml EtOH |
| | Leucine | 5.0 | 2.5 | 500 ml Water |
| | Tween 80 | 1.0 | 0.5 | |
| 5 | Melatonin | 70.0 | 35.0 | |
| | Lactose | 8.0 | 4.0 | 500 ml EtOH |
| | Glycine | 10.0 | 5.0 | 500 ml Water |
| | Leucine | 10.0 | 5.0 | |
| | Tween 80 | 2.0 | 1.0 | |
| 6 | Melatonin | 70.0 | 35.0 | |
| | Lactose | 24.0 | 12.0 | 500 ml EtOH |
| | Leucine | 5.0 | 2.5 | 500 ml Water |
| | Tween 80 | 1.0 | 0.5 | |
| 7 | Melatonin | 80.0 | 40.0 | |
| | Lactose | 16.0 | 8.0 | 500 ml EtOH |
| | Leucine | 3.0 | 1.5 | 500 ml Water |
| | Tween 80 | 1.0 | 0.5 | |
| 8 | Melatonin | 80.0 | 40.0 | |
| | Mannitol | 16.0 | 8.0 | 500 ml EtOH |
| | Leucine | 3.0 | 1.5 | 500 ml Water |
| | Tween 80 | 1.0 | 0.5 | |
| 9 | Melatonin | 50.0 | 25.0 | |
| | Lactose | 39.0 | 19.5 | 500 ml EtOH |
| | Leucine | 10.0 | 5.0 | 500 ml Water |
| | Tween 80 | 1.0 | 0.5 | |
| 10 | Melatonin | 50.0 | 25.0 | |
| | Mannitol | 39.0 | 19.5 | 500 ml EtOH |
| | Leucine | 10.0 | 5.0 | 500 ml Water |
| | Tween 80 | 1.0 | 0.5 | |
| 11 | Melatonin | 50.0 | 25.0 | |
| | Mannitol | 39.0 | 19.5 | 500 ml EtOH |
| | Leucine | 10.0 | 5.0 | 500 ml Water |
| | Poloxamer 188 | 1.0 | 0.5 | |
| 12 | Melatonin | 60.0 | 30.0 | |
| | Leucine | 39.0 | 19.5 | 500 ml EtOH |
| | Poloxamer 188 | 1.0 | 0.5 | 500 ml Water |
| 13 | Melatonin | 80.0 | 40.0 | |
| | Mannitol | 10.0 | 5.0 | 500 ml EtOH |
| | Leucine | 9.0 | 4.5 | 500 ml Water |
| | Poloxamer 188 | 1.0 | 0.5 | |

### CHARACTERISATION OF THE POWDER FOR RECONSTITUTION

The powders obtained were characterised in terms of dry particle size using a Sympatec Helos light-scattering appliance that analyses the particles size according to the Fraunhofer theory, fitted with a Rodos disperser.

The instrument was suitably calibrated with reference material -Sic-F1200'08, Sympatec GmbH, System-Partikel-Tecnik- and prepared according to the instructions provided in the instrument's user manual.

Following appropriate cleaning before analysis, a quantity of powder for each batch manufactured was analysed without performing any preliminary preparation on the sample. The dispersion gas used was compressed air suitably purified of particles and oil residues. The analysis mode defined therefore envisaged observing the following parameters relative to the sample, powder disperser and light scattering analyser.

Sample
- size: approximately 100 mg
- analysis feeding procedure: with a spatula
- sample pre-treatments: none

Rodos disperser
- Model M ID-NR 230 V/Hz 24Va
- Dispersion pressure: 3 bar

Light scattering analyser
- Model: Helos
- Analysis method: Fraunhofer
- Software version: Windox 4.0
- Analysis lens: R3 (0.5-175 µm)
- Minimum optical concentration: 1%
- Analysis activation threshold: minimum optical concentration detectable 1% for max 30 seconds of time and with at least 100 ms of sample exposure.

All the analyses were conducted in an environment with a controlled temperature and humidity corresponding to 25°C and 50% RH relative humidity.

As far as the characterisation of the melatonin content and corresponding degradation products are concerned, a suitable HPLC analysis method was used.

The analytical method is characterised by the following parameters:
Analysis column: Zorbax Sb-Aq C18, 150mm I.D. 4.6 µm
Column temperature: 25°C
Mobile phase: A: 0.018M phosphate buffer
(2.45 g/l KH2PO4 in MilliQ water at pH 3.0 for H3PO4)
B: Acetonitrile

| | | |
|---|---|---|
| Isocratic elution: | A:80% | B: 20% |
| Flow rate: | 1.5 ml/min | |
| Wavelength: | 225 nm | |
| Injection volume: | 5 µL | |
| Melatonin retention time: | 2.3 min | |
| Analysis time: | 5 mins | |

The instrument used for analysis was an Agilent model 1100 HPLC column with a diode array detector, model G1315B.

The samples of each powder to be analysed were obtained by dissolving 10 mg of powder in 20 ml of the mobile phase prepared for analysis.

The results for melatonin content and particle size are presented in Table 2.

With reference to the particle size of the powders manufactured it was considered acceptable to obtain melatonin powder particles with limit values of 90% the dimensional distribution (X90) of less than 100µm and volume mean diameter (VMD) values of less than 50 µm.

With reference to the melatonin content of the powders produced, it is considered acceptable for the melatonin content measured in the powders produced to be from 95% to 105% of the expected value, according to the prepared composition.

**Table 2**

| **Example** | **VMD (µm)** | **X₉₀ (µm)** | **Melatonin content (%)** |
|---|---|---|---|
| 1 (comp.) | 35.5 | 81.2 | 100.9 |
| 2 (comp.) | 63.3 | 152.3 | 99.0 |
| 3 | 28.3 | 59.2 | 102.0 |
| 4 | 10.8 | 23.3 | 100.0 |
| 5 | 13.0 | 24.2 | 99.3 |
| 6 | 9.7 | 18.9 | 101.1 |
| 7 | 10.2 | 20.0 | 99.2 |
| 8 | 8.2 | 15.5 | 99.0 |
| 9 | 5.5 | 10.1 | 101.6 |
| 10 | 5.7 | 10.3 | 102.7 |
| 11 | 3.7 | 7.1 | 100.5 |
| 12 | 3.0 | 5.5 | 101.4 |
| 13 | 10.5 | 17.9 | 101.8 |

### SOLUBILITY OF THE POWDER FOR RECONSTITUTION

In order to evaluate the solubility of the powdered melatonin formulations a series of melatonin powder samples was prepared by dissolving different quantities in the same volume of 3 solutions containing respectively:
- Purified water
- Water and PEG-400 (90/10)
- Water and PEG-400 (80/20)
- Water and PEG-400 (75/25; 70/30; 60/40; 50/50) -example 11 and 13 only-
- Water and Ethanol (90/10; 80/20; 75/25; 70/30) - example 11 and 13 only-
- Water and PEG-200 (75/25)
- Water and PEG-350 (75/25)

Following the preparation of a saturate solution of the powders obtained, the same solution was filtered with a 0.22 mm membrane and the filtered solution obtained was analysed by HPLC according to the method described previously.

The results are indicated in Table 3.

In particular table 3a shows the solubility values of melatonin roe material and the compositions of examples 1-13 in pure water and in water/PEG-400 mixture with different water/PEG ratios. Table 3b shows the solubility values of melatonin row material and the composition of the examples 11 and 13 in pure water and in water/ethanol mixture with different water/ethanol ratios.

Table 3c shows the solubility values of melatonin row material and the composition of the examples 11 and 13 in water/PEG mixture in ratio 75/25 with different type of PEG (PEG-200, PEG-350, PEG-400.

The data reported do not show a change in the solubility of the melatonin if used as a micronized crystalline raw material or after formulation in a powder form obtained by spray drying.

The example shows that a relationship exists between the maximum quantity of melatonin that can be dissolved and the amount of PEG-400 used.

Dissolution can be complete if the relationship between the amount of melatonin to be dissolved (expressed in mg) and the volume of PEG-400 used (expressed in ml) does not exceed the ratio 50/1.

**Table 3a**

| **Solubility mg/ml** | **Water** | **Water-PEG-400 ratio** | | | | | |
|---|---|---|---|---|---|---|---|
| | | **90-10** | **80-20** | **70-30** | **65-35** | **60-40** | **50-50** |
| **Composition Example** | | | | | | | |
| Melatonin raw material | 1.8 | 5.0 | 11.1 | - | - | - | - |
| 1 (comp.) | 2.0 | 4.7 | 10.7 | - | - | - | - |
| 2 (comp.) | 2.0 | 5.1 | 11.3 | - | - | - | - |
| 3 | 2.1 | 5.1 | 11.1 | - | - | - | - |
| 4 | 2.8 | 5.4 | 11.0 | - | - | - | - |
| 5 | 1.8 | 4.7 | 10.5 | - | - | - | - |
| 6 | 1.7 | 4.6 | 10.5 | - | - | - | - |
| 7 | 1.7 | 4.8 | 10.1 | - | - | - | - |
| 8 | 1.9 | 5.0 | 11.9 | - | - | - | - |
| 9 | 2.0 | 5.2 | 11.8 | - | - | - | - |
| 10 | nd | nd | 10.1 | - | - | - | - |
| 11 | 1.8 | 5.0 | 11.5 | 22.7 | 30.0 | 31.5 | 31.9 |
| 12 | 1.8 | 5.1 | 11.2 | - | - | - | - |
| 13 | 1.7 | 5.1 | 10.0 | 18.4 | 25.8 | 29.4 | 22.5 |

**Table 3b**

| **Solubility mg/ml** | **Water** | **Water-Ethanol ratio** | | | |
|---|---|---|---|---|---|
| | | **90-10** | **80-20** | **75-25** | **70-30** |
| **Composition Example** | | | | | |
| Melatonin raw material | 1,7 | 3.7 | 8.2 | 14.2 | 19.7 |
| 11 | 1,8 | 2.5 | 5.4 | 13.7 | 13.9 |
| 13 | 1,7 | 2.5 | 5.0 | 7.7 | 11.9 |

**Table 3c**

| **Solubility mg/ml** | **Water-PEG 75/25** | | |
|---|---|---|---|
| | **PEG 200** | **PEG 350** | **PEG 400** |
| **Composition Example** | | | |
| Melatonin raw material | 10.5 | 16.9 | 14.5 |
| 11 | 10.5 | 13.3 | 15.1 |
| 13 | 10.7 | 11.4 | 14.2 |

### DISSOLUTION SPEED OF THE POWDER FOR RECONSTITUTION

In order to document the difference in dissolution speed of formulated melatonin compared to non-formulated melatonin, 30 mg of pure melatonin and 60 mg of formulation 10 containing 30 mg of melatonin, were dissolved in 3 ml of a solvent mixture of water/PEG-400 (80/20). The entire test was conducted in temperature conditions of 25°C.

After 1 minute of manual stirring the two solutions were left to settle for 1 minute.

The visual inspection of the two preparations after 1 minute showed the presence of undissolved particulate in the case of the non-formulated melatonin and it presented on the other hand a clear solution in the case of the preparation containing the example 10 formulation.

After 1 minute of sedimentation, the supernatant solution was sampled by taking 1 ml of it and analysing it according to the HPLC method described previously.

The analysis revealed a concentration of melatonin present in the supernatant solution derived from the dissolution of the melatonin raw material of 7.1 mg/ml.

In the case, however, of the solution obtained from the powder of example 10, the concentration of melatonin present in the supernatant solution was equal to 10.2 mg/ml, corresponding to a complete melatonin dissolution.

After 10 minutes of stirring, the solution of melatonin raw material had a concentration of 7.9 mg/ml.

The test performed clearly showed that the pure melatonin was not compatible with the making of an extemporaneous preparation for administration by injection.

### STABILITY OF THE PREPARATION FOR INJECTION

A test was conducted to evaluate the stability of a melatonin solution obtained using the example 10 formulation as a comparison with the stability of a melatonin solution obtained from the pure raw material (melatonin raw material).

60 mg of example 10 formulation melatonin were dissolved in 3 ml of water/PEG-400 (80/20) solvent mixture.

In the same way 30 mg of melatonin raw material were dissolved in a volume of 3 ml of the same solvent composition.

For both the solutions obtained the dissolved melatonin content was initially measured by means of the HPLC method described previously.

This content was considered equivalent to 100% of the active substance present in the solution. The solutions were then stored at a temperature of 4°C and 25°C and analysed after 9 and 24 days.

The results obtained are indicated in Table 4.

The results show that melatonin raw material has the tendency to lose content in solution if stored at a low temperature.

This content loss can be attributed to the possible recrystallization of melatonin with precipitation.

In the case of the solution obtained from the dissolution of the example 10 formulation, this loss of content is not seen.

**Table 4**

| **Time (days)** | **4°C** | | **25°C** | |
|---|---|---|---|---|
| | **Melatonin raw material** | **Example 10** | **Melatonin raw material** | **Example 10** |
| 0 | 100.0 | 100.0 | 100.0 | 100.0 |
| 9 | 98.9 | 100.0 | 100.0 | 100.0 |
| 24 | 98.3 | 100.0 | 99.5 | 99.5 |

### STABILITY OF THE POWDER FOR RECONSTITUTION

To confirm the stability of the preparations of melatonin powder a stability study was conducted on the bulk powders of the example 9 and 10 formulations.

The powders were packaged in heat-sealed aluminium bags and stored for 6 months in conditions corresponding to 4°C or 25°C and 60% relative humidity.

Periodically, a sample of both formulations was taken and analysed to evaluate their :
- Dimensional distribution of the particles according to the method described previously
- Active substance content according to the HPLC method described previously
- Residual water content using a Coulometric Karl Fischer titrator, model C20X Mettler Toledo.

The results obtained are presented in Tables 5, 6, 7 and 8.

The analysis of the stability data collected for the two formulations studied made it possible to demonstrate the excellent stability of both formulations from a chemical and a physical point of view.

As far as the active substance content is concerned, this did not deteriorate significantly.

In the same way, the particle size characteristics of the powders produced were not altered for the entire duration of the study, in the same way as the moisture content of the two formulations also remains essentially unchanged.

It is therefore possible to conclude that the stability of melatonin in formulations of this type is guaranteed for up to 6 months.

**Table 5**

| **Formulation 9 Storage: 4°C** | | | | |
|---|---|---|---|---|
| | **T0** | **T=1 month** | **T=3 months** | **T=6 months** |
| Content (%) | 101.6 | 101.6 | 101.9 | 100.2 |
| X90 (µm) | 10.1 | 10.9 | 10.1 | 10.7 |
| VMD (µm) | 5.5 | 5.7 | 5.5 | 5.6 |
| Water content (%) | 1.0 | 1.0 | 1.0 | 1.0 |

**Table 6**

| **Formulation 10 Storage: 4°C** | | | | |
|---|---|---|---|---|
| | **T0** | **T=1 month** | **T=3 months** | **T=6 months** |
| Content (%) | 102.7 | 103.7 | 103.5 | 101.8 |
| X90 (µm) | 10.3 | 10.6 | 10.7 | 10.8 |
| VMD (µm) | 5.7 | 5.7 | 5.7 | 5.9 |
| Water content (%) | 0.9 | 0.8 | 0.9 | 0.9 |

**Table 7**

| **Formulation 9 Storage: 25°C/60% RH** | | | | |
|---|---|---|---|---|
| | **T0** | **T=1 month** | **T=3 months** | **T=6 months** |
| Content (%) | 101.6 | 102.0 | 100.4 | 99.9 |
| X90 (µm) | 10.1 | 10.9 | 11.0 | 11.3 |
| VMD (µm) | 5.5 | 5.8 | 5.7 | 5.9 |
| Water content (%) | 1.0 | 0.9 | 1.0 | 0.9 |

**Table 8**

| **Formulation 10 Storage: 25°C/60% RH** | | | | |
|---|---|---|---|---|
| | **T0** | **T=1 month** | **T=3 months** | **T=6 months** |
| Content (%) | 102.7 | 103.1 | 102.2 | 101.2 |
| X90 (µm) | 10.3 | 10.5 | 11.2 | 5.4 |
| VMD (µm) | 5.7 | 5.9 | 6.0 | 11.3 |
| | | | | 6.0 |
| Water content (%) | 0.9 | 1.0 | 0.9 | 0.9 |

As demonstrated by the tests, the powder for reconstitution according to the present invention presents good morphological characteristics, i.e. it is stable over time in terms of melatonin content.

Furthermore, the powder is easily soluble in an aqueous medium, thereby obtaining melatonin solutions with high concentrations and that are substantially devoid of undissolved particles. The powder also presents a high dissolution speed, such as to permit the preparation of extemporaneous solutions for injection over a short timeframe.

In particular, the tests conducted on examples 9 and 10 show how the powder for reconstitution according to the present invention, compared to the reference powders of examples 1 and 2 or to the melatonin raw material (non-formulated), is readily soluble in a mixture of water and PEG thereby obtaining melatonin solutions with concentrations suitable for administration in patients with cerebral infarction, in particular suitable for administration in newborns, thanks to the high concentrations achieved.

The solutions for injection obtained from dissolution of the powder for reconstitution according to the present invention are also stable after their preparation, thereby allowing use of the same even several days after preparation.

### OSMOLALITY OF SOLUTIONS FOR ADMINISTRATION

Following the concept of administering a solution with controlled properties in terms of pH and osmolality raw melatonin and the formulation of example 13 can be dissolved in compatible Water/PEG 400 solvent mixtures leading to different administrable solutions at different dosage for a fixed volume of delivery of 5 ml.

The results are presented in the tables that follow:
Melatonin raw material

| Water/PEG 400 ratio | Melatonin conc. in solution (mg/ml) | Volume of injection (ml) | Dose of melatonin (mg) | pH | mOsm/Kg |
|---|---|---|---|---|---|
| 90/10 | 5 | 5 | 25 | 5.2 | 335 |
| 85/15 | 7 | 5 | 35 | 5.2 | 530 |
| 80/20 | 10 | 5 | 50 | 5.2 | 754 |
| 75/25 | 14 | 5 | 70 | 5.2 | 1014 |
| 70/30 | 18 | 5 | 90 | 5.2 | 1310 |

Formulation of example 13

| Water/PEG 400 ratio | Melatonin conc. in solution (mg/ml) | Volume of injection (ml) | Dose of melatonin (mg) | pH | mOsm/Kg |
|---|---|---|---|---|---|
| 90/10 | 5 | 5 | 25 | 5.2 | 344 |
| 85/15 | 7 | 5 | 35 | 5.2 | 542 |
| 80/20 | 10 | 5 | 50 | 5.2 | 773 |
| 75/25 | 14 | 5 | 70 | 5.2 | 1042 |
| 70/30 | 18 | 5 | 90 | 5.2 | 1350 |

### RECTAL PREPARATION

In order to document the possibility of preparing a hydrophilic melatonin gel for rectal administration, 50 mg of pure melatonin or 62,5 mg of formulation 13 containing 50 mg of melatonin, were dissolved in 5 ml of a solvent mixture of water/PEG-400 (75/25).

The entire preparation was conducted in temperature conditions of 25°C, without additional heating. Following complete dissolution in the water/PEG 400 solvent mixture of melatonin or formulation 13, 100 mg of Carboxymethilcellulose (CMC) were gradually added in the two solutions and kept under continuous agitation for 20 minutes. At the end of this time a clear gel was formed.

## Claims

1. A powder for use as a medicament, **characterised in that** it comprises melatonin in an amounts from 35 to 90% in weight, at least one water soluble excipient in an amount from 5 to 60% in weight wherein said soluble excipient comprises leucine and at least one water soluble surfactant in an amount from 0.5 to 5% of the total weight of the powder, said powder having an X90 of less than 100 µm and a volume mean diameter VDM of less than 50 µm; and wherein said powder is obtained by spray drying from a solution comprising melatonin.

2. The powder for use according to claim 1, **characterised in that** it comprises particles in which melatonin, a water soluble excipient and a water soluble surfactant are present.

3. The powder for use according to one or more of the previous claims, **characterised in that** said water soluble surfactant is chosen from the group consisting of nonionic surfactants.

4. A pharmaceutical composition for use as a medicament in the form of a solution **characterised in that** it is obtained by dissolving a powder for use according to one or more of claims 1- 3, in a mixture of water and polyalkylene glycol, in which melatonin is present in an amount from 3 to 30 mg/ml and the polyalkylene glycol is present in an amount from 5 to 40% of the total volume of the liquid used.

5. The composition for use according to claim 4, **characterised in that** said glycol is polyethylene glycol (PEG) with a molecular weight from 200 to 600.

6. The composition for use according to claim 4 or 5, **characterised in that** a thickening or gelling agent is present in an amount from 0,5 to 50% w/v of the preparation, said thickening or gelling agent is chosen from the group consisting of: carboxymethylcellulose (CMC), hydroxypropylmethylcellulose (HPMC), hydroxyethylcellulose, hydroxypropylcellulose, microcrystalline cellulose (Avicel), chitosan, Sodium Alginate, Alginic acid, carrageenan, Guar gum, Gelatin, Hyrpomellose, Polyvinylpirrolidone (PVP), Poloxamers, Polyethylene glycols (MW > 600).

7. The composition according to one or more of claims 4-6, for use in the treatment of neonatal cerebral infarction.

8. The composition according to one or more of claims 4-6, for use in the treatment or prevention of perinatal asphyxia.

9. The composition according to one or more of claims 4-6, for use in the treatment or sleep disorders in a pediatric patient.

10. The composition according to one or more of claims 4-6, for use in the treatment of sleep disorders in Autism Spectrum Disorders (ASD)

11. The composition according to one or more of claims 4-6, for use in preanesthesia.

12. A composition for use as a medicament by oral administration comprising the composition according to one or more of claims 5-7 and breast feed milk.

13. A kit for the extemporaneous preparation of a solution, said kit comprising a powder for use according to one or more of claims 1-4, and a liquid medium comprising H₂O and polyalkylene glycol in an amount from 5 to 40% of the total volume of the liquid used

## Patentansprüche

1. Pulver zur Verwendung als ein Arzneimittel, **dadurch gekennzeichnet, dass** es Melatonin in einer Menge von 35 bis 90 Gewichtsprozent, mindestens einen wasserlöslichen Hilfsstoff in einer Menge von 5 bis 60 Gewichtsprozent, wobei der wasserlösliche Hilfsstoff Leucin aufweist, und mindestens ein wasserlösliches Tensid in einer Menge von 0,5 bis 5 % des Gesamtgewichts des Pulvers aufweist, wobei das Pulver einen X90-Wert von weniger als 100 µm und einen gemittelten Volumendurchmesser VDM von weniger als 50 µm aufweist; und wobei das Pulver durch Sprühtrocknen aus einer Melatonin aufweisenden Lösung erhalten wird.

2. Pulver zur Verwendung nach Anspruch 1, **dadurch gekennzeichnet, dass** es Partikel aufweist, in denen Melatonin, ein wasserlöslicher Hilfsstoff und ein wasserlösliches Tensid vorhanden sind.

3. Pulver zur Verwendung nach einem oder mehreren der vorhergehenden Ansprüche, **dadurch gekennzeichnet, dass** das wasserlösliche Tensid aus der Gruppe bestehend aus nichtionischen Tensiden ausgewählt ist.

4. Pharmazeutische Zusammensetzung zur Verwendung als ein Arzneimittel in Form einer Lösung, **dadurch gekennzeichnet, dass** sie durch Auflösen eines Pulvers zur Verwendung nach einem oder mehreren der Ansprüche 1 bis 3 in einem Gemisch aus Wasser und Polyalkylenglykol erhalten wird, in welchem Melatonin in einer Menge von 3 bis 30 mg/ml und das Polyalkylenglykol in einer Menge von 5 bis 40 % des Gesamtvolumens der verwendeten Flüssigkeit vorhanden ist.

5. Zusammensetzung zur Verwendung nach Anspruch 4, **dadurch gekennzeichnet, dass** das Glykol Polyethylenglykol (PEG) mit einem Molekulargewicht von 200 bis 600 ist.

6. Zusammensetzung zur Verwendung nach Anspruch 4 oder 5, **dadurch gekennzeichnet, dass** ein Verdickungs- oder Geliermittel in einer Menge von 0,5 bis 50 % Massenkonzentration der Zubereitung vorhanden ist, wobei das Verdickungs- oder Geliermittel ausgewählt ist aus der Gruppe bestehend aus: Carboxymethylcellulose (CMC), Hydroxypropylmethylcellulose (HPMC), Hydroxyethylcellulose, Hydroxypropylcellulose, mikrokristalliner Cellulose (Avicel), Chitosan, Natriumalginat, Alginsäure, Carrageen, Guargummi, Gelatine, Hypromellose, Polyvinylpyrrolidon (PVP), Poloxameren, Polyethylenglycolen (Molekulargewicht > 600).

7. Zusammensetzung nach einem oder mehreren der Ansprüche 4 bis 6 zur Verwendung bei der Behandlung von Hirninfarkten bei Neugeborenen.

8. Zusammensetzung nach einem oder mehreren der Ansprüche 4 bis 6 zur Verwendung bei der Behandlung oder Verhinderung von perinataler Asphyxie.

9. Zusammensetzung nach einem oder mehreren der Ansprüche 4 bis 6 zur Verwendung bei der Behandlung von Schlafstörungen bei einem Patienten im Kindesalter.

10. Zusammensetzung nach einem oder mehreren der Ansprüche 4 bis 6 zur Verwendung bei der Behandlung von Schlafstörungen bei Autismus-Spektrum-Störungen (ASD).

11. Zusammensetzung nach einem oder mehreren der Ansprüche 4 bis 6 zur Verwendung in der Präanästhesie.

12. Zusammensetzung zur Verwendung als ein Arzneimittel zur oralen Verabreichung, aufweisend die Zusammensetzung nach einem oder mehreren der Ansprüche 5 bis 7 und Muttermilch.

13. Kit zur Rezepturherstellung einer Lösung, wobei das Kit ein Pulver zur Verwendung nach einem oder mehreren der Ansprüche 1 bis 4 und ein flüssiges Medium aufweist, das H₂O und Polyalkylenglykol in einer Menge von 5 bis 40 % des Gesamtvolumens der verwendeten Flüssigkeit aufweist.

## Revendications

1. Poudre pour utilisation comme médicament, **caractérisée en ce qu'**elle comprend de la mélatonine en une quantité de 35 à 90 % en poids, au moins un excipient soluble dans l'eau en une quantité de 5 à 60 % en poids, dans laquelle ledit excipient soluble comprend de la leucine, et au moins un tensioactif soluble dans l'eau en une quantité de 0,5 à 5 % du poids total de la poudre, ladite poudre ayant un X90 inférieur à 100 µm et un diamètre moyen en volume VDM inférieur à 50 µm ; et dans laquelle ladite poudre est obtenue par séchage par pulvérisation à partir d'une solution comprenant de la mélatonine.

2. Poudre pour utilisation selon la revendication 1, **caractérisée en ce qu'**elle comprend des particules dans lesquelles sont présents de la mélatonine, un excipient soluble dans l'eau et un tensioactif soluble dans l'eau.

3. Poudre pour utilisation selon une ou plusieurs des revendications précédentes, **caractérisée en ce que** ledit tensioactif soluble dans l'eau est choisi dans le groupe constitué par les tensioactifs non ioniques.

4. Composition pharmaceutique pour utilisation comme médicament sous la forme d'une solution, **caractérisée en ce qu'**elle est obtenue par dissolution d'une poudre pour utilisation selon une ou plusieurs des revendications 1 à 3, dans un mélange d'eau et de polyalkylèneglycol, dans laquelle la mélatonine est présente en une quantité de 3 à 30 mg/ml et le polyalkylèneglycol est présent en une quantité de 5 à 40 % du volume total du liquide utilisé.

5. Composition pour utilisation selon la revendication 4, **caractérisée en ce que** ledit glycol est le polyéthylèneglycol (PEG) ayant un poids moléculaire de 200 à 600.

6. Composition pour utilisation selon la revendication 4 ou 5, **caractérisée en ce qu'**un agent épaississant ou gélifiant est présent en une quantité de 0,5 à 50 % p/v de la préparation, ledit agent épaississant ou gélifiant est choisi dans le groupe constitué par : carboxyméthylcellulose (CMC), hydroxypropylméthylcellulose (HPMC), hydroxyéthylcellulose, hydroxypropylcellulose, cellulose microcristalline (Avicel), chitosane, alginate de sodium, acide alginique, carraghénane, gomme de guar, gélatine, hypromellose, polyvinylpyrrolidone (PVP), poloxamères, polyéthylèneglycols (MW > 600).

7. Composition selon une ou plusieurs des revendications 4 à 6, pour utilisation dans le traitement de l'infarctus cérébral néonatal.

8. Composition selon une ou plusieurs des revendications 4 à 6, pour utilisation dans le traitement ou la prévention de l'asphyxie périnatale.

9. Composition selon une ou plusieurs des revendications 4 à 6, pour utilisation dans le traitement de troubles du sommeil chez un patient pédiatrique.

10. Composition selon une ou plusieurs des revendications 4 à 6, pour utilisation dans le traitement de troubles du sommeil dans les troubles du spectre autistique (ASD).

11. Composition selon une ou plusieurs des revendications 4 à 6, pour utilisation en préanesthésie.

12. Composition pour utilisation comme médicament par administration orale, comprenant la composition selon une ou plusieurs des revendications 5 à 7 et du lait d'allaitement.

13. Kit pour la préparation extemporanée d'une solution, ledit kit comprenant une poudre pour utilisation selon une ou plusieurs des revendications 1 à 4, et un milieu liquide comprenant H₂O et polyalkylèneglycol en une quantité de 5 à 40 % du volume total du liquide utilisé
